# EUROPEAN PATENT APPLICATION

(11) **EP 0 781 537 A1**
(43) Date of publication of application: **02.07.1997**
(21) Application number: 95120553.3
(22) Date of filing: 27.12.1995
(51) Int. Cl.: A61F 13/00

(54) **Disposable absorbent article**

(71) Applicant: THE PROCTER & GAMBLE COMPANY, Cincinnati, Ohio 45202 (US)
(72) Inventor: Spielmann, Hartmut M., D-74599 Michelbach an der Luecke (DE)
(74) Representative: Hirsch, Uwe Thomas

(57) **Abstract**

The present invention relates to an absorbent article comprising a topsheet (1) backsheet (2) and core (3) wherein said core comprises at least two depressed areas forming channels (5).

## Description

### Technical Field of the Invention

The present invention relates to disposable absorbent articles such as diapers or sanitary napkins which exhibit improved rate of fluid transfer from the topsheet to the core.

### Background of the Invention

Sanitary articles such as sanitary napkins, baby diapers, absorbent inserts, and absorbent adult incontinence articles are well-known in the art. Typically all these articles comprise a wearer facing surface and a garment facing surface. The wearer facing surface receives liquids and bodily discharges, such as menses which are to be absorbed onto a topsheet and then transfers them to an absorbent structure or core where they are stored.

Well-known topsheets described in the art include non-woven fabrics, woven fabrics and films. Woven- or non-woven fabrics are made of fibers which, by their nature provide apertures of varying and changing size depending on the selected direction for liquid transport through them. Films are often made of polymeric material and are rendered permeable by aperturing. These apertures are typically engineered to provide certain characteristics such as directional fluid transport and can vary in shape and size. The walls of the apertures define the amount of extension-if any beyond the plane of the film thickness and the direction of such extensions and are typically provided in the shape of a funnel. In this manner the topsheet can accommodate varying amounts and varying rates of fluid deposition and transport the discharges to the absorbent core.

In order to achieve maximum fluid transfer from the topsheet to the core, the topsheet is typically joined to the core. In addition to guaranteeing direct and intimate contact between the topsheet and core, thereby facilitating fluid transfer, the joining of the topsheet to the core also ensures product stability and core integrity, particularly during use.

A number of different methods may be employed to join the topsheet to the core. One such method involves the use of adhesive. The adhesive is typically applied onto the core as a spray or in spirals such that the core is essentially completely covered by the adhesive, prior to placement of the topsheet upon the core. The use of joining methods, adhesive in particular however, results in a number of problems. The topsheet tends to exhibit an increased sticky feeling, which is highly undesirable to the consumer. Furthermore, the fluid transfer performance of the topsheet is also detrimentally affected. This is due to the reduction in the surface area available for fluid transfer caused by the blockage of the topsheet apertures by the adhesive.

Thus, it is an aim of the present invention to provide an absorbent article having an acceptable fluid transfer profile from the topsheet to the core by placing the topsheet and core in intimate contact with one another in combination with providing a topsheet which exhibits a reduced in-use sticky feeling.

It has now been found that this problem may be addressed by joining the topsheet to the core only at certain specific areas. At these specific areas the core material is compressed to form a channel and the topsheet is joined to the core material in the depressed area. It is believed that the existence of channels results in tension being exerted across the topsheet such that intimate and direct contact between the core and topsheet is achieved and maintained. Joining is only required at areas of depression of the core, thus reducing the amount of joining material required. Furthermore, in addition to a reduction in the amount of joining material used which is of economical significance, the present invention also reduces the in-use sticky feeling of the topsheet.

A further advantage of absorbent articles comprising the channels of the present invention is that the channels also promote the rate of fluid transfer from the topsheet to the absorbent core.

The use of channels in absorbent articles to fulfill various functions has been previously described in the art. For example US 3 575 174 which discloses a sanitary napkin (flat or curved) which is maintained in a shaped configuration by deep embossed channels impressed through the cover and into the core to compression bond the two components. The napkin is constructed so as to be maintained comfortably in direct and intimate contact with the body of the wearer in order to effectively utilise its absorbing capacity.

Similarly, US 4 392 862 relates to absorptive devices having improved body contacting surfaces. The devices comprise a fluid permeable facing element, a fluid permeable support element, an absorbent core and an impervious backsheet which are superimposed upon one another. The document discloses that the facing element is too weak in nature to be utilised alone and is thus affixed to the support element by spaced apart regions of bonding forming an assembly which is then superimposed onto the absorbent core. The document also describes that the four superimposed elements can be united into a unitary article by any means such as hot melt adhesive about the margins of the device.

Sanitary napkins which minimise the probability of staining or side leakage by using embossed channels located adjacent to the longitudinal edges of the napkin are described in US 3 575 174. The channels are activated by the users thighs, so that the sides of the napkin fold up and form an occlusive container. The embossed channels are formed by compressing the layers of the pad a sufficient amount to remain intact throughout the use of the pad.

US 4 041 951 relates to absorptive devices such as disposable diapers, which present a dry surface feel to the user. The article comprises a planar absorbent layer placed between a moisture pervious topsheet and a moisture impervious backing sheet. The topsheet comprises a multiplicity of depressed areas which intimately contact the uppermost surface of the absorbent layer. The non depressed areas are contacted with the users skin. The accompanying figures show that the topsheet and absorbent layer are not in contact other than at the depressed areas. US 4 624 666 discloses the use of an impressed channel in a napkin to improve fluid transport properties and provide a cover which has clean and dry properties.

US 4 781 710 relates to absorbent pads having improved liquid distribution and retention. The pads comprise a densification pattern, wherein relatively low density tuft regions are separated and surrounded by channels. The channels comprise storage regions and transport regions. The pad comprises a topsheet, a backsheet and an absorbent pad located between the top- and backsheet, preferably in-between layers of tissue. The top- and backsheet preferably extend beyond the absorbent core and are affixed together along the edges. Alternatively, the topsheet may be indirectly attached to the backsheet, by affixing the topsheet to the intermediate members.

None of the identified prior art documents recognise or address the problem of reducing the bonding between the topsheet and core, whilst maintaining direct contact between the topsheet and core. Furthermore, although channels are known in the art, their particular application to solve the problem underlying the present invention has not been described.

### Summary of the Invention

The present invention is a disposable absorbent article comprising a liquid pervious topsheet, a backsheet and an absorbent core intermediate said topsheet and said backsheet, said topsheet, said backsheet and said core, each having a wearer facing surface and a garment facing surface,
said topsheet and said backsheet being joined to one another at the periphery of said absorbent article,
said core having at least two depressed areas forming channels, each of said depressed areas preferably having a depth of at least 25% of the thickness of said core, each of said depressed areas forming a strain region which extends around said depressed area without reaching the periphery of said core or another strain region, and
said garment facing surface of said topsheet being permanently joined to the wearer facing surface of said core in said strain region.

### Brief Description of the Drawings

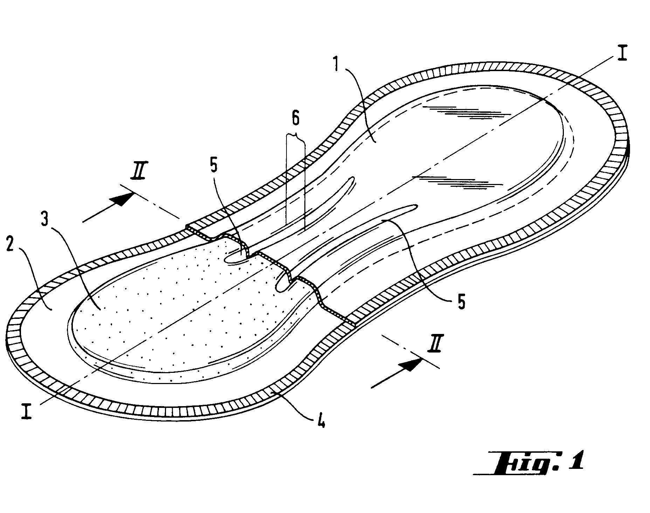

Fig. 1 is a top and perspective view of a preferred embodiment of an absorbent article of the present invention

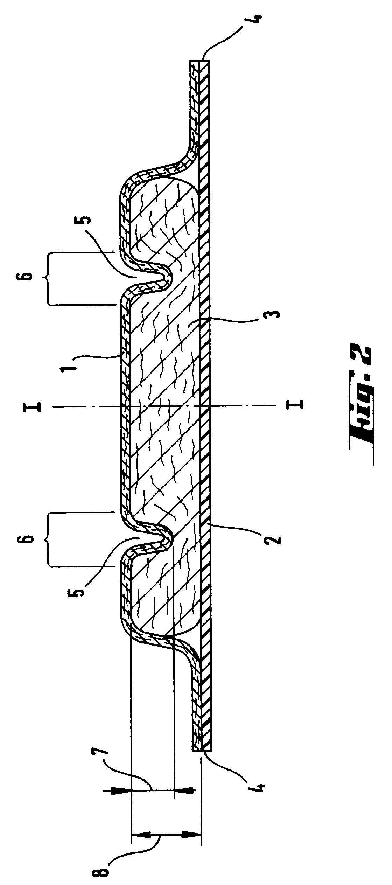

Fig. 2 is a partial cross sectional view of the absorbent article taken on line II-II of Fig. 1.

### Detailed Description of the Invention

The present invention relates to absorbent disposable articles such as sanitary napkins, baby diapers, incontinence products and panty liners. The absorbent articles of the present invention comprise the essential features of a liquid pervious topsheet (1), a backsheet (2) and an absorbent core (3) intermediate the topsheet and the backsheet. The topsheet, backsheet and core each have a wearer facing surface and a garment facing surface. The garment facing surface of the topsheet and the wearer facing surface of the backsheet are joined to one another at the periphery (4) of said absorbent article.

### Absorbent core

According to the present invention, the absorbent core (3) can include the following components: (a) an optional primary fluid distribution layer preferably together with a secondary optional fluid distribution layer; (b) a fluid storage layer; (c) an optional fibrous ("dusting") layer underlying the storage layer; and (d) other optional components. According to the present invention the absorbent core may have any thickness depending on the end use envisioned. In a preferred embodiment of the present invention wherein the absorbent article is a sanitary napkin, the core may have a thickness of from 2mm to 20mm, preferably from 4mm to 17mm, most preferably from 6mm to 12mm.

### a Primary/Secondary Fluid Distribution Layer

One optional component of the absorbent core according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilised. The fluid distribution layers can be comprised of any material typical for such distribution layers. In particular fibrous layers maintain the capillaries between fibers even when wet are useful as distribution layers.

### b Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials usually referred to as "hydrogel", "superabsorbent", hydrocolloid'' materials in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross-linked, partially neutralised, polymeric gelling material. This material forms a hydrogel upon contact with water. Such polymer materials can be prepared from polymerizable, unsaturated, acid-containing monomers which are well known in the art.

Suitable carriers include materials which are conventionally utilised in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins and panty liners.

An embodiment of the absorbent structure made according to the present invention may comprise multiple layers comprises a double layer tissue laminate formed by folding the tissue onto itself. These layers can be joined to each other for example by adhesive or by mechanical interlocking or by hydrogen bridge bands. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

### c Optional Fibrous ("Dusting") Layer

An optional component for inclusion in the absorbent core according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

### d Other Optional Components of the absorbent structure

The absorbent core according to the present invention can include other optional components normally present in absorbent webs. For example, a reinforcing scrim can be positioned within the respective layers, or between the respective layers, of the absorbent core. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

Another component which can be included in the absorbent core according to the invention and preferably is provided close to or as part of the primary or secondary fluid distribution layer are odour control agents. Active carbon coated or other odour control agents, in particular suitable zeolite or clay materials, are optionally incorporated in the absorbent structure. These components can be incorporated in any desired form but often are included as discrete particles.

### Absorbent Core Channels (1)

According to the present invention an essential feature of the absorbent core is that it comprises at least two depressed areas, which form channels (5) As used herein the term 'depressed area' refers to an area of the absorbent core having a depth (7) less than the thickness Fig. (8) of the core in its unstressed position or configuration under ambient pressure.

Each channel has a depth of at least 25% of the thickness of said core, preferably at least 75% more preferably at least 95%, most preferably at least 99% of the thickness of the core. The channels may have varying depth depending on the end use intended. For example, thin absorbent products such as panty liners wherein the core is typically 6 to 10mm thick, will have channels having a depth of typically from 9.6mm to 6.1mm. Thicker products however, such as thick sanitary napkins which have a thickness of typically from 11 to 20mm will accordingly have channels of greater depth. Preferably within the same product the depressed areas will also have substanially identical depth.

According to the present invention each depressed area of a channel (5) forms a strain region (6). The term strain region as used herein refers to an area which extends around said depressed area without reaching the periphery of said core or another strain region. Preferably said strain region extends a distance not more than 45%, preferably not more than 25%, most preferably not more than 15% of the shortest distance between an adjacent channel or between a channel and the periphery of said core, whichever is the closer. Typically this distance is not more than 50%, preferably not more than 25% of the maximum width of the channel. In a preferred embodiment said strain region does not extend beyond the depressed area.

The channels of the present invention may be of any size and may have any configuration such as spherical, oblong or rectangular. Preferably, the channels are elongated and extend longitudinally along the length of the core and have a similar shape to that of the circumference of the absorbent article.

The channels may also be located at any position on the core. In a preferred embodiment of the present invention wherein the absorbent article is utilised as a panty liner or sanitary napkin, the channels are preferably positioned closer to the periphery than the centre of the core and are more preferably substantially parallel with the periphery of the core or the absorbent article. More preferably said channels are located in the crotch region of the core. According to the present invention preferably at least two of the channels are diametrically opposed about the longitudinal/vertical plane (I-I) of symmetry in the crotch region.

The channels of the absorbent core of the present invention may be formed by any methods known in the art. Suitable methods include channel formation using embossing (heat, cold or chilled presses) or quilting.

### The topsheet

According to the present invention the absorbent article comprises as an esssential component a topsheet The topsheet may comprise a single layer or a multiplicity of layers. In a preferred embodiment the topsheet comprises a first layer which provides the user facing surface of the topsheet and a second layer between the first layer and the absorbent structure/core.

The topsheet as a whole and hence each layer individually needs to be compliant, soft feeling, and non-irritating to the wearers skin. It also can have elastic characteristics allowing it to be stretched in one or two directions. According to the present invention the topsheet may be formed from any of the materials available for this purpose and known in the art, such as woven and non woven fabrics and films. In a preferred embodiment of the present invention at least one of the layers, preferably the upper layer, of the topsheet comprises a liquid permeable apertured polymeric film. Preferably, the upper layer is provided by a film material having apertures which are provided to facilitate liquid transport from the wearer facing surface towards the absorbent structure. If present the lower layer of the topsheet is selected from preferably a non woven layer, an apertured film or an airlaid tissue.

According to the present invention it is an essential feature that the garment facing surface of said topsheet is permanently joined to the wearer facing surface of said absorbent core at said strain region. Preferably, the topsheet is only joined to the core at said strain region and the periphery of said topsheet and core, more preferably the topsheet is only permanently joined to the core in said strain regions. Similarly, if the topsheet consists of a multiplicity of layers, all adjacent layers of the topsheet are joined to one another at the location of the corresponding said strain region.

As used herein the term joined refers to any means known in the art for affixing two adjacent layers of material. Suitable joining means include adhesion, fusion bonding, ultra sonic bonding, stitching, heat (e.g. crimping) and/or pressure bonds, or dynamic mechanical bonds According to a preferred embodiment of the present invention the preferred means of joining is adhesive. Suitable adhesives include non pressure sensitive and cold adhesives available from Findlay and Fullers. The adhesive may be applied by any means known in the art such as spiral application, slot coating, spraying, curtain coating, control coating and printing. The application of the adhesive to the core/ topsheet is achieved by applying the adhesive to the topsheet or the core using one of the aforementioned means in the strain region.

The joining between the garment facing surface of the topsheet and the wearer facing surface must be such that the layers are permanently affixed to one another. Thus, it is not essential that the layers are joined over the entire strain region. Hence, partial and intermittent joining may be utilised and is indeed desirable from an economic standpoint.

According to the present invention the joining of the garment facing surface of said topsheet to said wearer facing surface of said core at said channels in the above described manner is such that at least 50%, preferably at least 90%, most preferably at least 95% of the wearer facing surface of said core is in intimate and direct contact with the garment facing surface of said topsheet. Most preferably, the topsheet and core are substantially in intimate and direct contact when said article is in its unstressed position or configuration at areas where said topsheet is superimposed on said core.

### Backsheet

The backsheet primarily prevents the extrudes absorbed and contained in the absorbent structure from wetting articles that contact the absorbent product such as underpants, pants, pyjamas and undergarments. The backsheet is preferably impervious to liquids (e.g. menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet also can have elastic characteristics allowing it to stretch in one or two directions.

The backsheet typically extends across the whole of the absorbent structure and can extend into and form part of or all of the preferred sideflaps, side wrapping elements or wings.

The backsheet can comprise a woven or nonwoven material, polymeric films such as thermoplastic films of polyethylene or polypropylene, or composite materials such as a film-coated nonwoven material. Preferably, the backsheet is a polyethylene film having a thickness of from about 0.012 mm (0.5 mil) to about 0.051 mm (2.0 mils).

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matt finished to provide a more clothlike appearance.

According to the present invention the absorbent article may find utility in sanitary napkins, panty liners, adult incontinence products and baby diapers. In particular the present invention finds application in sanitary napkins and panty liners.

## Claims

1. A disposable absorbent article comprising a liquid pervious topsheet, a backsheet and an absorbent core intermediate said topsheet and said backsheet, said topsheet, said backsheet and said core each having a wearer facing surface and a garment facing surface,
said topsheet and said backsheet being joined to one another at the periphery of said absorbent article,
said core having at least two depressed areas forming channels (5), each of said channels preferably having a depth of at least 25% of the thickness of said core (3), each of said depressed areas forming a strain region (6) which extends around said depressed area without reaching the periphery (4) of said core (3) or another strain region (6), and
said garment facing surface of said topsheet being permanently joined to said wearer facing surface of said core in said strain region (6).

2. A disposable absorbent article according to claim 1, wherein at least 50 % of the wearer facing surface of said core (3) is in intimate and direct contact with the garment facing surface of said topsheet when said article is in its unstressed configuration.

3. A disposable absorbent article according to claim 2, wherein at least 90 % of the wearer facing surface of said core (3) is in intimate and direct contact with the garment facing surface of said topsheet when said article is is in its unstressed configuration.

4. A disposable absorbent article according to any of the preceding claims, wherein said garment facing surface of the topsheet (1) and said wearer facing surface of the core (2) are joined to one another in said strain region (6) by a means selected from adhesion, fusion bonding, and ultra sonic bonding.

5. A disposable absorbent article according to any one of the preceding claims, wherein said garment facing surface of the topsheet (1) and said wearer facing surface of the core (3) are permanently joined to one another in said strain region by adhesive.

6. A disposable absorbent article according to any one of the preceding claims, wherein said strain region (6) extends a distance not more than 45% of the shortest distance between adjacent channels or between a channel and the periphery of said core.

7. A disposable absorbent article according to any one of the preceding claims, wherein each channel has a maximum width and said strain region extends a distance not more than 50% of the maximum width of said channel.

8. A disposable absorbent article according to any one of the preceding claims, wherein said topsheet (1) comprises at least two layers, wherein at least one layer is a liquid permeable apertured polymeric film.

9. A disposable absorbent article according to claim 8, wherein said topsheet (1) comprises two layers, a first layer and a second layer, wherein the wearer facing surface of said second layer and the garment facing surface of said first layer are permanently joined to one another at said strain region (6).

10. A disposable absorbent article according to any one of the preceding claims, wherein said absorbent article is a sanitary napkin or a panty liner.
